# EUROPEAN PATENT APPLICATION

(11) **EP 2 958 036 A1**
(43) Date of publication of application: **23.12.2015**
(21) Application number: 15169548.3
(22) Date of filing: 28.05.2015
(51) Int. Cl.: G06F 19/00

(54) **A SYSTEM FOR STORAGE AND ACCESS TO DATA RELATIVE TO A LIVING BEING**

(30) Priority: 28.05.2014 IT VR20140151
(71) Applicant: Zelig - Serviços de Consultadoria Lda, 9000 051 Funchal Madeira (PT)
(72) Inventor: De Nisco, Sonia, 41019 Soliera (Modena) (IT)
(74) Representative: Anselmi, Davide

(57) **Abstract**

A system (1) for storage of and access to data (2) relative to a living being, comprising a basic electronic apparatus (3) for connection to an information technology network (4) configured for receiving the data (2) relative to the living being and for loading it on the information technology network (4). The system (1) is configured for associating the data (2) loaded on the information technology network (4) with a corresponding identification code (10) for protecting the data (2).

More specifically, the system (1) comprises an electronic device (11) configured for storing the identification code (10) and for communicating with the basic electronic apparatus (3). This electronic device (11) is configured for receiving a signal (15) requesting access to the identification code (10), and for providing the identification code (10) if the electronic device (11) receives a corresponding enabling signal (16) for access to the identification code (10).

## Description

This invention relates to a system and a method for storage and access to data relative to a living being.

More specifically, this invention operates on data relating to a person or to an animal. Preferably, this invention operates on data of a medical type (for example, data relating to medical treatments, allergies, blood group, incompatible medicines, passed operations, etc.), but could be applied for any other type of data (relating to the fashion, sports sectors etc.).

In addition, this invention relates to a system for loading data on a server connected to an information technology network (preferably the Internet) using an electronic apparatus (for example, smartphone, handheld device, tablet PC, etc.) and giving access to the data using this electronic apparatus or by another electronic apparatus especially in emergency situations. Special systems for storage of and access to data are not known in the prior art and, in general, the data relating to a person is usually kept in special hardcopy files stored by the person.

In any case, there are systems which enable storage of the data in a device or on a server connected to an information technology network. In this way, a user can access the data displaying it on an electronic apparatus. However, since this data is confidential it is often stored under special names or protected using passwords known only by the user to whom the data relates.

Consequently, in situations of extreme danger (for example, a road accident ) where the user is unable (for example, because they unconscious) to provide the details for identifying the data stored, it is not possible to recover and access the data. It should be noted that the access to the data could be of fundamental importance for saving the life of the person who is injured or in a serious condition.

This known technology therefore has several drawbacks. More specifically, the main drawback is that, under certain situations, it is not possible for a third person to access the data of the user.

Consequently, it is not possible to know certain data relating to the user in real time which might be useful for some types of intervention or to prevent further injury to the user.

In this situation, the aim of this invention is to provide a system and a method for storage of and access to the data relative to a living being which overcomes the above-mentioned drawbacks.

More specifically, the aim of this invention is to provide a system and a method for storage of and access to the data relative to a living being which allows the data to be stored securely and to facilitate access to the data at least in situations of particular danger.

Another aim of this invention to provide a system and a method for storage of and access to the data relative to a living being which allows the data to be accessed in a quick and easy fashion.

Lastly, the aim of this invention to provide a system and a method for storage of and access to the data relative to a living being which allows the data to be accessed without the authorisation of the living being.

The aims indicated are substantially achieved by a system and method for storage and access to data relative to a living being as described in the accompanying claims.

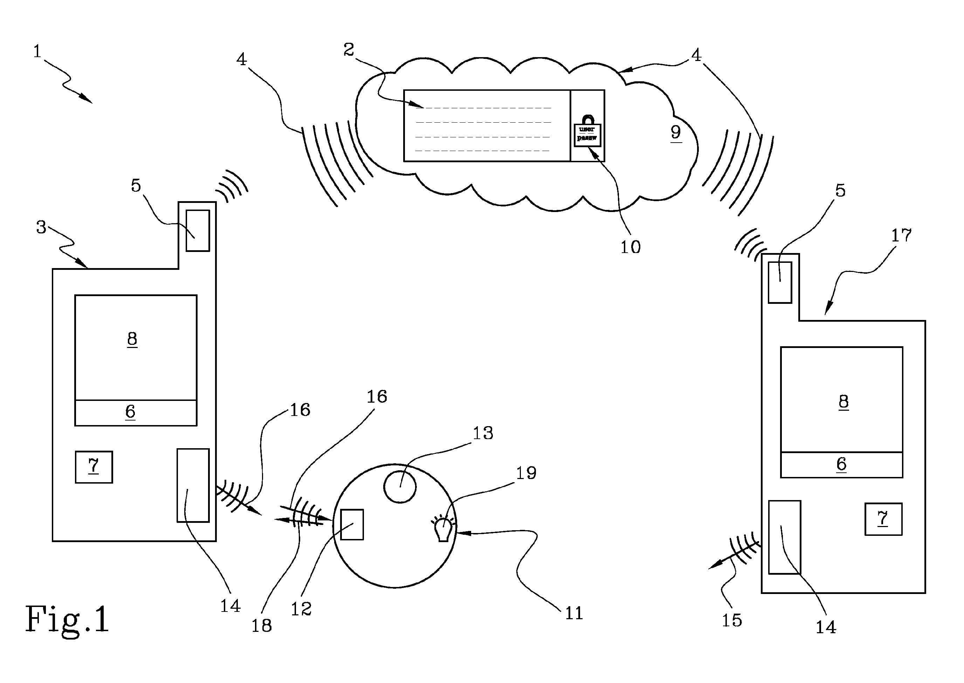

Further characteristic features and advantages of this invention will emerge more clearly from the detailed description of several preferred, but not exclusive embodiments of a system and a method for storage of and access to data relative to a living being illustrated in Figure 1 which shows a schematic view of the system for storage of and access to data relative to a living being according to this invention.

With reference to the above-mentioned drawing, the reference numeral 1 denotes in its entirety a system for storage of and access to data 2 relative to a living being according to this invention.

As already mentioned, the data 2 preferably relates to a person or to an animal. Reference will be made mainly in the following description to the data 2 relating to a person, but without restricting the scope of the invention towards the data 2 relating to an animal.

More specifically, the system 1 comprises an electronic apparatus 3 for connection to an information technology network 4 configured for receiving the data 2 relative to the living being and for loading it on the information technology network 4 using a relative first communication module 5. Preferably, the system 1 comprises a plurality of sensors for measuring biometric parameters of a person operatively connected with the basic electronic apparatus 3 for sending to the latter corresponding signals for measuring the biometric parameters measured. The sensors for measuring the biometric parameters are, during use, connected to or interacting with the body of the patient in such a way as to measure biometric parameters (heart rate, blood pressure, insulin, etc.). The basic electronic apparatus 3 is configured for receiving the signals for measuring the biometric parameters and for loading them on the information technology network 4 using the communication module 5.

More in detail, the basic electronic connecting apparatus 3 comprises means 6 for interaction with a user for transmission (entering) of the data 2 relative to the living being. Preferably, the interaction means 6 comprise a keyboard and/or a touch-screen and/or any other interaction system 1 not explicitly mentioned herein.

Moreover, the basic electronic apparatus 3 preferably comprises a storage unit 7 designed for storing the data 2 in the basic electronic apparatus 3. More specifically, the storage unit 7 may be configured for storing the data 2 in the basic electronic apparatus 3 only temporarily (until the data 2 is loaded on the information technology network 4) or permanently so as to keep it stored directly on the basic electronic apparatus 3.

Moreover, the basic electronic apparatus 3 is configured for loading the data 2 on the information technology network 4 using a relative first communication module 5.

More specifically, the basic electronic apparatus 3 is operatively connected to the information technology network 4 for interacting with it. The first communication module 5 preferably comprises a transceiver antenna by which to load the data 2 on the information technology network 4 or by which to access it.

In any case, the basic electronic apparatus 3 is configured to interact with the information technology network 4 in a two-way fashion.

More specifically, the basic electronic connection apparatus 3 is further configured for accessing the data 2 loaded on the network (using the first communication module 5) to make it available to a user using a relative display interface 8. In detail, the communication interface comprises a display screen.

Moreover, the information technology network 4 preferably comprises at least one server 9 ("cloud" server) on which the data 2 relative to the living being is loaded and stored.

The information technology network 4 is preferably defined by the Internet network, but could be defined by any local information technology network 4 or by both networks.

Also, it should be noted that the basic electronic apparatus 3 preferably comprises a smartphone (as shown in Figure 1) and/or a handheld device and/or a computer and/or a tablet and/or any other basic electronic apparatus 3 not explicitly mentioned in this description but suitable to perform the above-mentioned operations.

In addition to the above, the system 1 is configured for associating the data 2 loaded on the information technology network 4 with a corresponding identification code for protecting the data 2. In other words, the data 2 relative to a living being which is loaded on the information technology network 4 is associated with a protection identification code 10 to prevent unauthorised accesses to the data 2.

Consequently, any signal requesting access to the data 2 which is sent to the information technology network 4 must also contain the identification code 10, in order to access the data 2.

Preferably, the basic electronic apparatus 3 is configured for storing the identification code 10 in such a way as to directly access the data 2 loaded on the information technology network 4.

The identification code 10 preferably comprises one or more alphanumeric sequences (for example, user name and password).

According to this invention, the system 1 comprises an electronic device 11 which in turn comprises a relative transceiver module 12. The electronic device 11 is connected to the basic electronic apparatus 3 to exchange data 2 with the latter. The electronic device 11 is external and movable in an independent manner relative to the body of the living being. In other words, the electronic device 11 is disconnected relative to the body of the living being. In yet other words, the electronic device 11 is portable by the living being outside the body (for example, it is held in the hand or in a pocket,...) The electronic device 11 preferably comprises a memory register 13, a CPU and a power supply battery. If necessary, the electronic device 11 also comprises a global position identification element (for example GPS). Preferably, the electronic device 11 is physically separated from basic electronic apparatus 3 and is configured for communicating with the latter in a wireless fashion using the transceiver module 12. In other words, the basic electronic apparatus 3 is configured for communicating with the electronic device 11 using a relative second communication module 14 which is operatively associated with the transceiver module 12. In yet other words, the electronic device 11 and the basic electronic apparatus 3 are configured for communicating with each other respectively through the transceiver module 12 and the second communication module 14.

Basically, the basic electronic apparatus 3 is configured for communicating with the electronic device 11 using a communication system 1 which is different from the communication system 1 used to exchange data 2 with the information technology network 4.

Moreover, the transceiver module 12 of the electronic device 11 and the second communication module 14 are configured for communicating with each other using data exchange systems 2 without previous coupling. Even more preferably, the transceiver module 12 of the electronic device 11 and the second communication module 14 are configured for communicating with each other using the Bluetooth standard 4.

Alternatively, the transceiver module 12 of the electronic device 11 and the second communication module 14 are configured for communicating with each other using systems configured for exchanging the data only by contact between the transceiver module 12 and the second module 14 (for example, NFC or RFID systems).

It should be noted that the electronic device 11 can preferably be associated with the collar of an animal in the case of data 2 relating to an animal.

Preferably, the transceiver module 12 of the electronic device 11 has a radius of action adjustable between 0 to 50 metres. Advantageously, in the case of data 2 relating to a person, the transceiver module 12 is configured to have a radius of action of between 0 and 5 cm.

Advantageously, in the case of data 2 relating to animals, the transceiver module 12 is configured to have a radius of action of between 0 and 50 metres since it is not always possible to move the basic electronic apparatus 3 close to the animal.

In addition, the electronic device 11 is configured for storing the identification code 10 on the relative memory register 13.

Moreover, the electronic device comprises a lighting element 19 (for example, LEDs) configured to signal the communication between the basic electronic apparatus 3 and the electronic device 11. More specifically, the lighting element 19 is configured for signalling the exchange of data in progress between the basic electronic apparatus 3 and the electronic device 11. For example, when a user moves towards the electronic device 11 with a smartphone (basic electronic apparatus 3) and the two are able to exchange information, the electronic device 11 activates the LED 19 indicating transmission of signal.

Preferably, the system 1 is configured for generating an identification code 10 as a function of a predetermined serial number associated with the electronic device 11.

It should also be noted that in the preferred embodiment the electronic device 11 is a "Beacon" device.

More specifically, the electronic device 11 is configured for:
- receiving a signal 15 requesting from the outside access to the identification code 10 using the transceiver module 12;
- providing the identification code 10 to the outside if the electronic device 11 receives a corresponding enabling signal 16 for access to the identification code 10.

More specifically, the signal 15 requesting access is generated by an external electronic apparatus 17 (having features equal to those of the basic electronic apparatus 3 described above) which requests the data 2 relative to the living being. This situation is generated when a third user wants to access the data 2 of the user using the relative external electronic apparatus 17.

In detail, the electronic device 11 is configured for sending to the basic electronic apparatus 3 the access request signal 15. The electronic device 11 is in turn configured for sending the access request signal 15 to the basic electronic apparatus 3.

Consequently, the basic electronic apparatus 3 is configured for:
- receiving the access request signal 15 from the electronic device 11;
- providing the information contained in the request signal to a user using the display interface 8;
- sending the enabling signal 16 to the electronic device 11 for enabling the access as a function of an interaction produced by the user with interaction means 6 of the basic electronic apparatus 3.

In other words, following receipt of the signal 15 requesting access, the electronic device 11 is configured for sending this request for access to the basic electronic apparatus 3 in such a way that the latter can make the request visible to the user.

Preferably, the information contained in the request signal is displayed on the basic electronic apparatus 3 using the screen of the display interface 8. At this point, the user interacts with the interaction means 6 (for example, using the keyboard or touchscreen) to grant authorisation or, at least, to provide access to the identification code 10 and hence to the data 2.

In other words, the interaction means 6 generate and send to the electronic device 11 the enabling signal 16 if the user has expressed the desire for access to the data 2 through these interaction means 6.

In that case, the electronic device 11 is configured for sending a response signal 18 to the external electronic apparatus 17 containing the access code.

In any case, the electronic device 11 is configured for providing the identification code 10 for a predetermined period of time after which the identification code 10 is no longer available.

Alternatively to what is described above, the request signal 15 for the identification code 10 also contains the information of the signal 16 for enabling access to the identification code 10 in such a way that the electronic device 11 directly provides the information code 10. In that case, the electronic device 11 is configured for receiving the request signal 15 and for sending the response signal 18 containing the identification code 10.

Advantageously, the latter situation occurs in relation to a small and predetermined number of external electronic apparatuses 17 (for example, belonging to a pre-authorised medical department) which have been, in advance, enabled for accessing the data 2 of the users.

In any case, the external electronic apparatus 17 may, after receiving the identification code 10, access the information technology network 4 (by means of the relative first communication module 5) for downloading the data 2 relative to the living being.

Moreover, the basic electronic apparatus 3 is configured to be associated with a plurality of electronic devices 11 each relating to a respective living being.

In addition, the basic electronic apparatus 3 is configured for calculating the distance of the electronic device 11 relative to it and for making it visible using the display interface 8. In that way, it is possible monitor the distance of one or more electronic devices 11 relative to the basic electronic apparatus 3.

It should also be noted that the electronic device 11 is configured for providing the identification code 10 only for a predetermined radius of action beyond which the identification code 10 is no longer available.

This invention also relates to a method for storage and access to data 2 relative to a living being. The method is derived directly from what is described above with regard to the system 1, which is here below incorporated in its entirety.

More specifically, the method comprises a first operating step of loading the data 2 on the information technology network 4 using the basic electronic apparatus 3 for connection to the information technology network 4. More specifically, the step for loading the data 2 comprises sending it to a server 9 (defining part of the information technology network 4) for storing it. Moreover, the method comprises associating the data 2 with a protection identification code 10 for access to the data 2. In other words, for access to the data 2 it is necessary for the signal 15 requesting the data 2 to contain the identification code 10.

Moreover, the method comprises a step for accessing the data 2 using the basic electronic apparatus 3 in such a way as to make it available to a user using the display interface 8. The basic electronic apparatus 3 contains the identification code 10 in such a way that the step for accessing the data 2 occurs without the need to perform requests for the identification code 10. According to this invention, the method comprises a step of storing the identification code 10 in the electronic device 11 operatively associated with the basic electronic connection apparatus 3. More specifically, the electronic device 11 is in communication with the basic electronic apparatus 3 using a predetermined radio transmission system 1.

Moreover, the method comprises a step for providing the identification code 10 to other electronic apparatuses which request it by using the electronic device 11. The step of providing the identification code 10 comprises the sub-steps of:
receiving a request for access to the identification code 10 from the external electronic apparatus 17 using the electronic device 11;
providing the identification code 10 if the electronic device 11 is enabled to allow access to the identification code 10.

More specifically, the sub-step of enabling the electronic device 11 to access the identification code 10 comprises a further sub-step requesting the enabling of the basic electronic apparatus 3 using the relative means 6 for interaction with the user. If the user the relative consent for enabling using the interaction means 6, the basic electronic apparatus 3 communicates to the electronic device 11 that the latter may release the identification code 10 to other electronic apparatuses.

Alternatively, the sub-step of enabling the electronic device 11 for access to the identification code 10 comprises a further sub-step of pre-associating the request for access to the identification code 10 for the enabling access to the identification code 10 in such a way as to directly obtain the access code.

Lastly, the method comprises a final step for accessing the data 2 archived on the information technology network 4 using the further electronic apparatus.

This invention also relates to a computer program for storage of and access to data 2 relative to a living being characterised in that the operations listed above in reference to the method are performed.

The present invention achieves the set aims.

Firstly, this system enables data relative to a living being to be stored in a cloud server in such a way that it is potentially available to anyone who has an electronic apparatus. In any case, access to the data requires an identification code which is stored in an electronic device separate from but associated with the electronic apparatus. Advantageously, in this way it is possible to access the data using a further electronic apparatus by a request for access which occurs using the electronic device.

In this way, the access to the data occurs in a secure manner and is available to whoever is authorised.

More specifically, some electronic apparatuses associated with groups of pre-authorised persons (for example, a medical group) already possess the authorisation to the identification code in such a way as to deal with particular emergency situations (for example, injury or states of unconsciousness of a user).

In addition, the system allows the distance of a plurality of electronic devices from the electronic apparatus to be localised in such a way as to know the position of living beings to whom a respective electronic device is associated.

It should also be noted that this invention is relatively easy to implement and that the cost of implementing the invention is relatively low.

## Claims

1. A system (1) for storage and access to data (2) relative to a living being comprising:
basic electronic apparatus (3) for connection to an information technology network (4) configured for receiving the data (2) relative to the living being and for loading it on the information technology network (4) using a relative first communication module (5); the basic electronic communication apparatus (3) being further configured for accessing the data (2) loaded on the network in such a way as to make it available to a user using a relative display interface (8);
the system (1) being configured for associating the data (2) loaded on the information technology network (4) with a corresponding identification code (10) for protecting the data (2);
**characterised in that** it comprises an electronic device (11) comprising a relative transceiver module (12) and configured for storing the identification code (10); the basic electronic apparatus (3) being configured for communicating with the electronic device (11) using a relative second communication module (14) which is operatively associated with the transceiver module; the electronic device (11) being external and movable independently of the body of the living being; the electronic device (11) being configured for:
- receiving a signal (15) requesting access to the identification code (10) from an external electronic device (17) using the transceiver module (12);
- providing the identification code (10) to the external electronic device (17) if the electronic device (11) receives a corresponding enabling signal (16) for access to the identification code (10).

2. The system (1) according to claim 1, **characterised in that** the electronic device (11) is configured for sending to the basic electronic apparatus (3) the access request signal (15); the electronic device (11) being configured for sending the access request signal (15) to the basic electronic apparatus (3); the basic electronic apparatus (3) being configured for:
- receiving the access request signal (15) from the electronic device (11);
- providing the information contained in the request signal (15) to a user using the display interface (8);
- sending the enabling signal (16) to the electronic device (11) for enabling the access as a function of an interaction produced by the user with interaction means (6) of the basic electronic apparatus (3).

3. The system (1) according to any one of the preceding claims, **characterised in that** the basic electronic apparatus (3) is configured for storing the identification code (10) in such a way as to directly access the data (2) loaded on the information technology network (4).

4. The system (1) according to any one of the preceding claims, **characterised in that** the request signal (15) to the identification code (10) also contains the information of the signal (16) for enabling access to the identification code (10) in such a way that the electronic device (11) directly provides the information code (10).

5. The system (1) according to any one of the preceding claims, **characterised in that** the electronic device (11) is configured for providing the identification code (10) for a predetermined interval of time.

6. The system (1) according to any one of the preceding claims, **characterised in that** the transceiver module (12) of the electronic device (11) has a radius of action adjustable between 0 and 50 metres.

7. The system (1) according to any one of the preceding claims, **characterised in that** transceiver module (12) of the electronic device (11) and the second communication module (14) are configured for communicating with each other using data exchange systems (2) without previous coupling.

8. The system (1) according claim 7, **characterised in that** transceiver module (12) of the electronic device (11) and the second communication module (14) are configured for communicating with each other using the Bluetooth standard (4).

9. The system (1) according to any one of the preceding claims, **characterised in that** the basic electronic apparatus (3) is configured for calculating the distance of the electronic device (11) relative to it.

10. The system (1) according to any one of the preceding claims, **characterised in that** the basic electronic apparatus (3) comprises a smartphone and/or a handheld device and/or a computer.

11. The system (1) according to any one of the preceding claims, **characterised in that** the electronic device (11) comprises a Beacon device.

12. The system (1) according to any one of the preceding claims, **characterised in that** it comprises a plurality of sensors for measuring biometric parameters of an individual operatively connected with the basic electronic apparatus (3) for sending to the latter corresponding signals for measuring the biometric parameters measured; the basic electronic apparatus (3) being configured for receiving the signals for measuring the biometric parameters and for loading them on the information technology network (4) using the communication module (5).

13. A method for storage and access to data (2) relative to a living being comprising the following operating steps:
loading the data (2) on an information technology network (4) using a basic electronic apparatus (3) for connection to the information technology network (4);
associating the data (2) with a protection identification code (10) for access to the data (2);
accessing the data (2) using the basic electronic apparatus (3) in such a way as to make it available to a user using a relative display interface (8); **characterised in that** it comprises the following operating steps:
storing the identification code (10) in an electronic device (11) operatively associated with the basic electronic connection apparatus (3) and outside and movable independently of the body of the living being;
providing the identification code (10) to other electronic apparatuses by using the electronic device (11); the step of providing the identification code (10) comprising the sub-steps of:
receiving a request for access to the identification code (10) from an external electronic device (17) using the electronic device (11);
providing the identification code (10) to the external electronic device (17) if the electronic device (11) is enabled to allow access to the identification code (10).

14. A program for processing means for storage and access to data (2) relative to a living being **characterised in that** the operations of the method according to claim 13 are performed.
